# EUROPÄISCHE PATENTSCHRIFT

(11) **EP 2 161 307 B1**
(45) Veröffentlichungstag und Bekanntmachung des Hinweises auf die Patenterteilung: **29.08.2012**
(21) Anmeldenummer: 09159961.3
(22) Anmeldetag: 12.05.2009
(51) Int. Cl.: C09B 48/00, C09B 67/22, G02B 5/22, G02F 1/1335

(54) **Fluorhaltige Chinacridone in Farbfiltern für LCD**
Chinacridones containing fluoride in colour filters for LCD
Chinacridone contenant du fluor dans des filtres colorés pour écran LCD

(30) Priorität: 24.05.2008 DE 102008025006
(43) Veröffentlichungstag der Anmeldung: 10.03.2010
(73) Patentinhaber: LANXESS Deutschland GmbH, 51369 Leverkusen (DE)
(72) Erfinder: Feldhues, Ulrich, 51465 Bergisch Gladbach (DE); Linke, Frank, 51069 Köln (DE); Michaelis, Stephan, 51519 Odenthal (DE); Pfuetzenreuter, Dirk, 51399 Burscheid (DE); Berneth, Horst, 51373 Leverkusen (DE); Rolf, Meinhard, 51373 Leverkusen (DE)

(56) Entgegenhaltungen:
- EP-A- 1 004 941
- WO-A-2005/017046
- WO-A-2006/003090
- GB-A- 1 339 936
- JP-B- 44 022 417
- JP-B- 44 022 418
- US-A1- 2005 011 403
- US-A1- 2006 044 561
- DATABASE WPI Week 200866 Thomson Scientific, London, GB; AN 2008-L25408 XP002562594 -& JP 2008 103476 A (TOYO INK MFG CO LTD) 1. Mai 2008 (2008-05-01)
- DATABASE WPI Week 199639 Thomson Scientific, London, GB; AN 1996-388739 XP002562595 -& JP 08 188772 A (TOYO INK MFG CO LTD) 23. Juli 1996 (1996-07-23)
- DATABASE WPI Week 199942 Thomson Scientific, London, GB; AN 1999-502479 XP002562596 -& JP 11 217514 A (TORAY IND INC) 10. August 1999 (1999-08-10)

## Beschreibung

Die Erfindung betrifft die Verwendung von speziellen Fluor-haltigen Chinacridonen in Farbfiltern für LCD (liquid crystal displays) sowie Präparationen solcher Farbmittel und ihre Verwendung zur Herstellung von Farbfiltern, die Farbfilter selbst sowie noch neue Chinacridone.

Farbfilter finden heute vornehmlich Anwendung in Flüssigkristallanzeigen, Flüssigkristallbildschirmen, Farbauflösungsgeräten und Sensoren. Ein bekanntes Beispiel sind die Flachbildschirme für Personalcomputer, Fernsehgeräte und Videokameras. Es gibt verschiedene Methoden zur Herstellung der Farbfilter, die sich sowohl in der Auftragung der Farben als auch der Erzeugung der Farbelementmuster der Basisfarben rot, grün und blau neben schwarz unterscheiden. Die Applikation bzw. Auftragung der Farben kann z. B. durch Einfärben einer Trägerschicht (z. B. Gelatine) mittels löslichen Farbstoffen oder Pigmenten ("Dye Method", "Dye Dispersion Method"), Siebdruck, Offsetdruck oder Tintenstrahldruck von Pigmentpasten, -präparationen oder -tinten, Elektrodeposition von Photolacken auf Basis von Farbstoffen oder Pigmenten sowie insbesondere mittels der Pigment-Dispersionsmethode, bei der Pigmente verwendet werden, die entweder in einem Polyimidharz ("nicht-photosensitive Polyimidmethode") oder in einem Photolack ("photosensitive Acrylmethode") dispergiert sind, erfolgen. Verbunden mit den genannten Verfahren kommt sowohl der drucktechnisch direkten Erzeugung der Farbelementmuster als auch der indirekten, photolithographischen Erzeugung Bedeutung zu, letztere insbesondere bei der o. g. Pigment-Dispersionsmethode. Die Technik der Pigment-Dispersionsmethode in Form der "nichtphotosensitiven Polyimidmethode" ist beispielsweise in JP-A-11-217514 (1998) offenbart.

Bei der Pigment-Dispersionsmethode nach dem Photolackverfahren liegen die farbgebenden Pigmente in einem UV-härtbaren Photolack fein verteilt (dispergiert) vor. Der Photolack besteht dabei neben dem Pigment im Allgemeinen aus den Komponenten Bindemittelharz, polymerisierbares Monomer, Photoinitiator sowie gegebenenfalls einem Lösungsmittel. Die Herstellung erfolgt z. B. derart, dass zunächst das Pigment in Form eines Konzentrates mit Lösungsmittel und gegebenenfalls Bindemittelharz feinteilig dispergiert wird und unmittelbar vor der Applikation zusammen mit dem Monomer und dem Photoinitiator sowie gegebenenfalls weiterer Komponenten eingestellt wird. Der pigmentierte Photolack wird auf das Substrat, z. B. Glas, gleichmäßig aufgetragen, z. B. mittels des sogenannten "spin-coating"-Verfahrens, vorgetrocknet, mittels der Photomaske mit UV-Licht belichtet, mittels einer in der Regel anorganisch alkalischen Lösung zu den gewünschten Farbelementmustern entwickelt, die Beschichtung gereinigt und gegebenenenfalls nachgehärtet. Dieser Prozess wird für jede Farbe wiederholt, in der Regel also dreimal für eine Trichromie z. B. in den Farben rot, grün und blau.

Die Vorteile bei der Verwendung von Pigmenten in Verbindung mit der Pigment-Dispersionsmethode liegen in der verbesserten Licht-, Feuchtigkeits- und Temperaturbeständigkeit der Farbfilter im Vergleich zu Farbstoff-basierten Beschichtungssystemen. Demgegenüber sind die Transparenz und Farbreinheit der Beschichtungen auf Basis von Pigmenten, unabhängig vom Beschichtungsverfahren, noch nicht zufriedenstellend. Insbesondere wenn verschiedene Pigmente in Mischung zur Nuancierung auf die gewünschten Farbortwerte im Photolack eingearbeitet werden, kommt es zu unerwünschten Brillanz- und Transparenzverlusten, so dass als Folge die Anzeigen bzw. Bildschirme (LCD) mit einem erhöhten Energieaufwand betrieben werden müssen.

In roten Farbfiltern werden häufig Pigmente vom Typ Perylentetracarbonsäurediimid wie C.I. Pigment Rot 179 bzw. Diketopyrrolopyrrol wie C.I. Pigment Rot 254 bzw. Anthrachinone wie C.I. Pigment Rot 177 verwendet.

In EP-A-1004941 werden Mischkristalle von Chinacridonen wie C.I. Pigment Violett 19 und C.I. Pigment Rot 122 und ihre Verwendung u. a. für Colorfilter beschrieben. Neben dem einzigen konkreten Mischkristall werden allgemein auch solche mit durch Fluor am Chinacridonkern substituierte Chinacridone erwähnt.

In WO 2006/003090 werden Arylamin-substituierte Chinacridone beschrieben, die für verschiedene Einsatzgebiete wie beispielsweise für LCD's verwendet werden.

In JP-A-2002348493 werden Mischungen von ggf. durch Fluor am Chinacridonkern substituierte Chinacridone und ihrer sulfonierten Derivate für Colorfilter offenbart. Diese Pigmenttypen zeichnen sich teilweise bereits durch gute Lichtechtheit und Farbstärke aus. Transparenz und Farbreinheit sind noch nicht zufriedenstellend. Im übrigen weist die Herstellung von Mischkristallen (feste Lösungen) häufig Reproduzierbarkeits-Probleme hinsichtlich der Qualität auf, die sich dann insbesondere auf Transparenz und Farbreinheit, aber auch auf Farbstärke und Lichtechtheit nachteilig auswirken können.

Die Aufgabe der vorliegenden Erfindung ist demnach die Bereitstellung von roten organischen Pigmenten und ihre Verwendung in roten Farbfiltern für LCD sowie Präparationen solcher organischer Pigmente, die diese Nachteile nicht aufweisen.

Gegenstand der Erfindung ist daher die Verwendung von Chinacridonen der Formel (I) 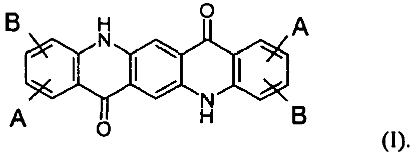 worin
A für einen durch ein oder mehrere Fluoratome substituierten organischen Rest steht,
B für H, F, Cl, Br oder für einen ggf. substituierten organischen Rest steht, der ggf. zusammen mit A einen Ring bilden kann,
in Farbfiltern für LCD.

Vorzugsweise enthalten die Verbindungen der Formel (I) keine Sulfogruppen.

Vorzugsweise steht B für Wasserstoff und A für C₁-C₈-Alkyl, C₁-C₈-Alkoxy ,Phenyl oder Phenoxy, das jeweils durch ein oder mehrere Fluoratome substituiert ist.

Ebenfalls vorzugsweise bilden A und B gemeinsam eine durch ein oder mehrere Fluor-Atome substituierte Brücke, die mit zwei benachbarten C-Atomen des Benzolrings der Formel (I) einen fünf-, sechs- oder siebengliedrigen Ring bildet, der carbocyclisch ist oder Heteroatome wie O, S oder N enthalten kann.

Geeignete durch Fluor substituierte C₁-C₈-Alkyl-Reste oder C₁-C₈-Alkoxy-Reste sind beispielsweise Methyl, Ethyl oder gegebenenfalls verzweigte Propyl-, Butyl-, Pentyl-, Hexyl- oder Octyl-Reste oder die entsprechenden Alkoxyreste, die mindestens ein Fluoratom tragen. Beispiele sind Fluormethyl, Trifluormethyl, 2-Fluorethyl, 2,2,2-Trifluorethyl, Pentafluorethyl, 2,2,3,3-Tetrafluorpropyl, Perfluorbutyl, Perfluoroctyl, insbesondere Perfluor-n-octyl und die entsprechenden Alkoxyreste.

Geeignete durch Fluor substituierte Phenyl- oder Phenoxyreste sind beispielsweise 2-, 3- oder 4-Fluorphenyl oder -phenoxy, 2,4- oder 3,4-Difluorphenyl oder -phenoxy, Pentafluorphenyl oder - phenoxy.

Geeignete ggf. substituierte organische Reste als mögliche Bedeutung von B sind vorzugsweise durch ein oder mehrere Fluoratome substituierte organische Reste, insbesondere solche, die unabhängig von A die gleiche Bedeutung wie der Rest A haben. Besonders bevorzugt sind die bevorzugten Bedeutungen von A, insbeondere Trifluormethyl.

Eine geeignete durch A und B gemeinsam gebildete Brücke ist beispielsweise

-O-CF₂-O- , -O-CF₂ CF₂-O-, OCHF-CHF-O- oder -O- CF₂ CF₂ CF₂-O-.

Bevorzugte Pigmente der Formeln (I) entsprechen den Formeln (II) bis (VIII).

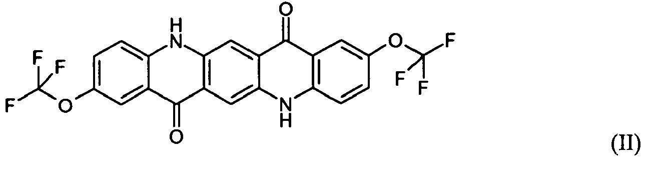

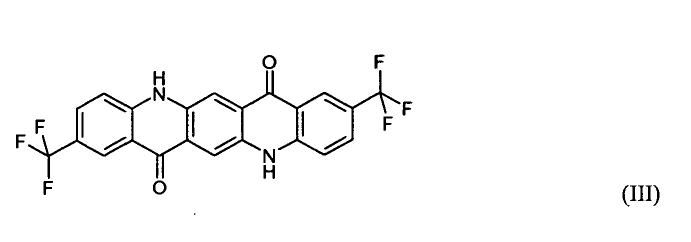

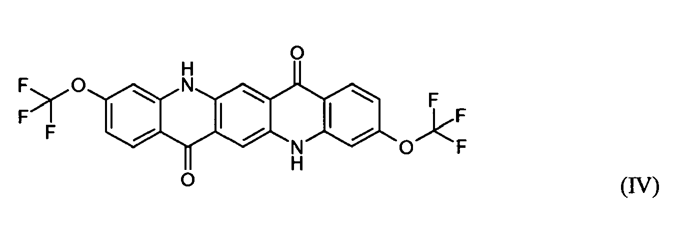

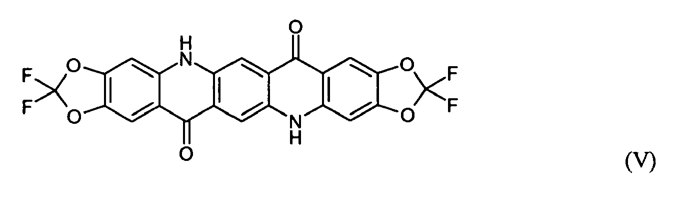

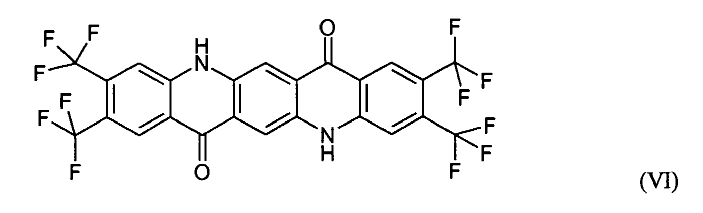

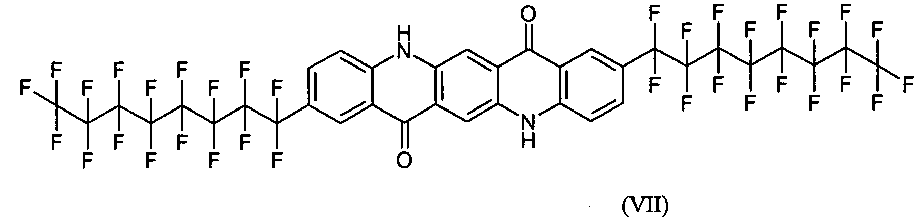

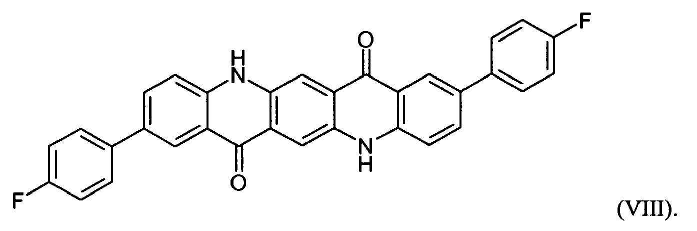

Besonders bevorzugt sind neue Pigmente der Formel (II), die im Röntgenbeugungsdiagramm (Cu-K_{α}-Strahlung) Linien bei folgenden d-Werten aufweisen (d: 5,30; d: 4,09; d: 3,69; d: 3,22) und in dieser Anmeldung als Modifikation A bezeichnet werden.

Ebenfalls besonders bevorzugt sind neue Pigmente der Formel (II), die im Röntgenbeugungsdiagramm (Cu-K_{α}-Strahlung) Linien bei folgenden d-Werten aufweisen (d: 4,22; d: 3,55; d: 3,31) und in dieser Anmeldung als Modifikation B bezeichnet werden.

Die Partikelgröße und Oberfläche der erfindungsgemäß verwendeten Pigmente kann eingestellt werden durch Methoden, die an sich bekannt sind und beispielsweise in US 6,068,695 aufgeführt sind, wie z.B. Salzknetung oder Kugelmahlung und/oder ggf. nachgelagerte Finishingschritte wie z.B. Temperungen in wässrigen, organischen oder wässrig/organischen Lösungsmitteln mit oder ohne Additivzusatz.

Die erfindungsgemäß verwendeten Pigmente besitzen vorzugsweise eine spezifische Oberfläche von 40 bis 200 m²/g, insbesondere von 60 bis 140 m²/g, ganz besonders bevorzugt von 70 bis 120 m²/g. Die Oberfläche wird nach DIN 66131 ermittelt: Bestimmung der spezifischen Oberfläche von Feststoffen durch Gasadsorption nach Brunauer, Emmett und Teller (B.E.T.)

Die erfindungsgemäß verwendeten Pigmente besitzen vorzugsweise eine Dispergierhärte von 10 bis 500, gemessen nach DIN 53775, Teil 7, wobei die Temperatur der Kaltwalzung 25°C und die Temperatur der Warmwalzung 150°C beträgt. Insbesonders bevorzugt verwendete Pigmente besitzen eine Dispergierhärte von 20 bis 250.

Die Dispergierhärte wird gemessen nach DIN 53 775, Teil 7, wobei die Temperatur der Kaltwalzung 25°C und die der Warmwalzung 150°C beträgt.

Sämtliche in dieser Anmeldung angegebenen Dispergierhärten wurden nach dieser modifizierten DIN-Vorschrift bestimmt.

Die erfindungsgemäß verwendeten Pigmente besitzen vorzugsweise eine Partikelgröße von (Längsachse im Transmissions-Elektronen-Mikroskop) von 10 bis 200 nm, insbesondere von 20 bis 100 nm. Bevorzugt besitzen die erfindungsgemäß verwendeten Pigmente eine enge Partikelgrößenverteilung mit einer relativen Standardabweichung (Standardabweichung/Partikelgröße) < 50%, insbesondere < 35%, besonders bevorzugt < 20%. Bevorzugt besitzen die erfindungsgemäß verwendeten Pigmente ein Längen- zu Breitenverhältnis von 5 : 1 bis 1 : 1, insbesondere von 3 : 1 bis 1 : 1, besonders bevorzugt von 2 : 1 bis 1,2 : 1.

Die erfindungsgemäß verwendeten Pigmente der Formel (I) können auch in Kombination mit anderen Pigmenten eingesetzt werden, beispielsweise zur Optimierung der optischen Eigenschaften der Farbfilter. Hinsichtlich der Auswahl anderer gegebenenfalls mitzuverwendender Pigmente besteht erfindungsgemäß keine Einschränkung. Es kommen sowohl anorganische als auch organische Pigmente in Frage.

Bevorzugte organische Pigmente sind z.B. solche der Monoazo-, Disazo-, verlackte Azo-, β-Naphthol-, Napthol AS-, Benzimidazolon-, Chinacridon-, Disazokondensations-, Azometallkomplex-, Isoindolin- und Isoindolinon-Reihe, ferner polycyclische Pigmente wie z.B. aus der Phthalocyanin-, Chinacridon- (soweit andere als die der Formel I), Perylen-, Perinon-, Thioindigo-, Anthrachinon-, Dioxazin-, Chinophthalon- und Diketopyrrolopyrrol-Reihe. Außerdem verlackte Farbstoffe wie Ca-, Mg- und Al-Lacke von sulfonsäure- oder carbonsäuregruppenhaltigen Farbstoffen. Ganz besonders bevorzugt wird der mit Melamin interkallierte Nickelkomplex der Azobarbitursäure als mitzuverwendendes Pigment beansprucht.

Beispiele für gegebenenfalls mitzuverwendende andere organische Pigmente, die im Color Index bekannt sind:
Color Index Pigment Yellow 12, 13, 14, 17, 20, 24, 74, 83, 86, 93, 94, 109, 110, 117, 125, 137, 138, 139, 147, 148, 150, 153, 154, 166, 173, 185,
Color Index Pigment Orange 13, 31, 36, 38, 40, 42, 43, 51, 55, 59, 61, 64, 65, 71, 72, 73,
Color Index Pigment Red 9, 97, 122, 123, 144, 149, 166, 168, 177, 179, 180, 192, 215, 216, 224, 254,272,
Color Index Pigment Green 7, 10, 36, 37, 45,
Color Index Pigment Blue 15, 15:1, 15:2, 15:3, 15:4, 15:6, 16,
Color Index Pigment Violett 19, 23.

Als weitere nicht aus dem Color Index bekannte Pigmente sind beispielsweise das aus DE102005033581, insbesondere Beispiel 2, bekannte Melamin interkallierte Nickelazobarbitursäurekomplex Pigment der Formel 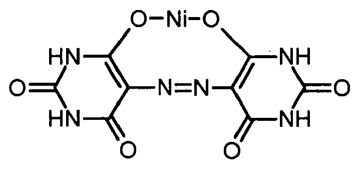 sowie seine tautomeren Formen zu nennen.

Ebenfalls sind sulfonierte Derivate der erfindungsgemäß verwendeten Pigmente der Formel (I) zu nennen.

Sofern "andere Pigmente" als die der Formel (I) zusätzlich mitverwendet werden, beträgt der Anteil von "Pigment" im obigen Sinne entsprechend der Formel (I) vorzugsweise 1-99 Gew.-%, insbesondere 20-80 Gew.-% bezogen auf die eingesetzte Gesamtmenge aller Pigmente.

Vorzugsweise mitzuverwendende Pigmente sind Pigment Rot 122, Pigment Rot 149, Pigment Rot 177, Pigment Rot 179, Pigment Rot 254, Pigment Violett 19, Pigment Gelb 138, Pigment Gelb 139, Pigment Gelb 150 oder ein mit Melamin interkalliertes Nickelazobarbitursäurekomplex-Pigment.

Besonders bevorzugt ist die Verwendung von Pigment-Mischungen enthaltend
- wenigstens ein Pigment der Formel (I)
- wenigstens ein weiteres Rotpigment aus der Gruppe C.I. Pigment Rot 122, C.I. Pigment Rot 149, C.I. Pigment Rot 177, C.I. Pigment Rot 179, C.I. Pigment Rot 254 und C.I. Pigment Violett 19
   in Farbfilter für LCD.

Diese bevorzugt einzusetzende Mischung enthält ggf. zusätzliche ein oder mehrere Gelbpigmente.

Die mitverwendeten Gelbpigmente bzw. Orangepigmente besitzen vorzugsweise eine Absorptionsbande im Bereich von 400 bis 520 nm.

Besonders enthält diese bevorzugte Mischung Gelbpigmente ausgewählt aus der Gruppe C.I. Pigment Gelb 138, C.I. Pigment Gelb 139, C.I. Pigment Gelb 150 und ein Melamin interkaliertes Nickelazobarbitursäurekomplex-Pigment.

Die Mischungen als solche sind ebenfalls Gegenstand dieser Erfindung.

Sofern als "andere Pigmente" gelbe oder orange Pigmente eingesetzt werden, beträgt der Anteil dieser gelben oder orangen "anderen Pigmente" vorzugsweise 1 bis 50 Gew.-%, insbesondere 5 - 30 Gew.-% bezogen auf die eingesetzte Gesamtmenge aller Pigmente.

Sofern als "andere Pigmente" rote Pigmente eingesetzt werden, beträgt der Anteil dieser roten "anderen Pigmente" vorzugsweise 1 bis 99 Gew.-%, insbesondere 20 - 80 Gew.-% bezogen auf die eingesetzte Gesamtmenge aller Pigmente.

Die mit den erfindungsgemäßen Pigmenten oder ihren Mischungen hergestellten Farbfilter zeichnen sich insbesondere durch hohe Farbreinheit und excellente Transparenz aus.

Ebenfalls bevorzugt werden die Pigmente der Formel (I) in Form einer Mischung verwendet, die neben der Formel (I) noch Gelbpigmente enthält. Vorzugsweise solche, die eine Absorptionsbande im Bereich von 400 bis 520 nm besitzen. Besonders enthält diese bevorzugte Mischung Gelbpigmente ausgewählt aus der Gruppe C.I. Pigment Gelb 138, C.I. Pigment Gelb 139, C.I. Pigment Gelb 150 und ein Melamin interkaliertes 1:1 1 Nickelazobarbitursäurekomplex-Pigment. Auch diese Mischung als solche ist ebenfalls Gegenstand dieser Erfindung.

Die erfindungsgemäße Verwendung der oben beschriebenen Pigmente bzw. Pigmentmischungen zur Herstellung von Farbfiltern für Flüssigkristallanzeigen sei im Folgenden am Beispiel der Pigment-Dispersionsmethode nach dem Photolackverfahren beschrieben.

Die erfindungsgemäße Verwendung der erfindungsgemäßen Pigmente zur Herstellung von Farbfiltern ist beispielsweise **dadurch gekennzeichnet, dass** das Pigment, gegebenenfalls mit einem Bindemittelharz und einem organischen Lösungsmittel, gegebenenfalls unter Zusatz eines Dispergiermittels, homogenisiert und anschließend kontinuierlich oder diskontinuierlich nasszerkleinert wird, insbesondere auf eine Teilchengröße nach Anzahl (elektronenmikroskopische Bestimmung) von 99,5 % <1000 nm, vorzugsweise 95 % <500 nm und insbesondere 90 % <200 nm.
Als Nasszerkleinerungsverfahren kommen beispielsweise Rührer- oder Dissolverdispergierung, Mahlen mittels Rührwerkskugel- oder -perlmühlen, Kneter, Walzenstuhl, Hochdruckhomogenisierung oder Ultraschalldispergierung in Frage.

Während der Dispergierbehandlung oder im Anschluss daran erfolgt die Zugabe von mindestens einem photohärtbaren Monomeren und einem Photoinitiator. Im Anschluss an die Dispergierung kann noch weiteres Bindemittelharz, Lösungsmittel oder für Photolacke übliche Zuschlagstoffe eingebracht werden, wie es für die gewünschte photosensitive Beschichtungsmitteleinstellung (Photolack) zur Herstellung der Farbfilter erforderlich ist. Im Rahmen dieser Erfindung wird unter Photolack eine Präparation verstanden, die wenigstens ein photohärtbares Monomer und einen Photoinitiator zusätzlich zum Pigment der Formel (I) enthält.

Als mögliche Dispergiermittel kommen allgemein handelsübliche wie beispielsweise polymere, ionogene oder nicht-ionogene Dispergiermittel bspw. auf Basis von Polycarbonsäuren oder Polysulfonsäuren, sowie Polyethylenoxid-Polypropylenoxid-Block-Copolymere in Betracht. Ferner können auch Derivate organischer Farbstoffe als Dispergiermittel oder Co-Dispergiermittel verwendet werden.

Bei der Herstellung der Farbfilter fallen daher "Zubereitungen" an, die bezogen auf die Zubereitung enthalten:
- wenigstens ein Chinacridon der Formel (I), welches im Sinne dieser Anmeldung als erfindungsgemäßes Pigment bezeichnet wird,
- gegebenenfalls ein oder mehrere andere Pigmente,
- gegebenenfalls ein Bindemittelharz,
- wenigstens ein organisches Lösungsmittel sowie
- gegebenenfalls ein Dispergiermittel.

In einer bevorzugten Ausführungsform enthält die Zubereitung (Angaben bezogen auf Zubereitung):

| | |
|---|---|
| 1 - 50 Gew.-% | wenigstens eines Chinacridons der Formel (I) |
| 0 - 50 Gew.-% | eines oder mehrerer anderer Pigmente |
| 0 - 20 Gew.-% | Bindemittelharz |
| 0 - 20 Gew.-% | Dispergiermittel |
| 10 - 94 Gew.-% | organisches Lösungsmittel. |

Die Beschichtung des Photolackes auf eine Platte zur Erzeugung der gefärbten Bildelementmuster kann entweder durch direkten oder indirekten Auftrag geschehen. Als Auftragsmethoden seien bspw. genannt: Ink-Jet, Roller-Coating, Spin-Coating, Spray-Coating, Dip-Coating und Air-Knife-Coating.

Als Platten kommen je nach Verwendung beispielsweise in Frage: transparente Gläser wie weiße oder blaue Glasplatte, mit Silikat beschichtete blaue Glasplatte, Kunstharzplatte oder -filme auf Basis von z.B. Polyester-, Polycarbonat-, Acryl-, oder Vinylchloridharz, ferner Metallplatten auf Basis von Aluminium, Kupfer, Nickel, oder Stahl sowie Keramikplatten oder Halbleiterplatten mit aufgebrachten photoelektrischen Transferelementen.

Die Auftragung erfolgt im Allgemeinen so, dass die Schichtdicke der erhaltenen photosensitiven Schicht bei 0,1 bis 10 µm liegt.

Im Anschluss an den Auftrag kann eine thermische Trocknung der Schicht erfolgen.

Die Belichtung erfolgt vorzugsweise, indem die photosensitive Schicht einem aktiven Lichtstrahl vorzugsweise in Form eines Bildmusters mittels Photomaske ausgesetzt wird. Hierdurch wird an den belichteten Stellen die Schicht gehärtet. Geeignete Lichtquellen sind z.B.: Hochdruck- und Ultrahochdruckquecksilberdampflampe, Xenon-, Metallhalogenid-, Fluoreszenzlampe sowie Laserstrahl im sichtbaren Bereich.

Durch die Entwicklung im Anschluss an die Belichtung wird der unbelichtete Teil der Beschichtung entfernt und man erhält die gewünschte Bildmusterform der Farbelemente. Übliche Entwicklungsmethoden umfassen das Besprühen mit oder Tauchen in wässrige alkalische Entwicklerlösung oder in ein organisches Lösungsmittel, das anorganische Alkali wie z.B. Natrium- oder Kaliumhydroxid, Natriummetasilikat oder organische Basen wie Monoethanolamin, Diethanolamin, Triethanolamin, Triethylamin oder deren Salze enthält.

Nach der Entwicklung erfolgt in der Regel eine thermische Nachtrocknung/-härtung der Bildmuster.

Als Bindemittelharze, die zusammen mit dem "Pigment" oder darauf basierender Pigmentpräparationen (also enthaltend Bindemittelharz und Pigment der Formel (I)) in Farbfiltern bzw. in den Zubereitungen zur Herstellung von Farbfiltern z.B. nach der Pigment-Dispersionsmethode, eingesetzt werden können, besteht erfindungsgemäß keine besondere Einschränkung, insbesondere kommen für die Anwendung in Farbfiltern an sich bekannte filmbildende Harze in Frage.

Beispielsweise kommen Bindemittelharze aus der Gruppe der Celluloseharze wie Carboxymethylhydroxyethylcellulose und Hydroxyethylcellulose, Acrylharze, Alkydharze, Melaminharze, Epoxidharze, Polyvinylalkohole, Polyvinylpyrrolidone, Polyamide, Polyamidimine und Polyimide in Frage.

Als Bindemittelharze kommen auch solche in Frage, die photopolymerisierbare, ungesättigte Bindungen enthalten. Die Bindemittelharze können beispielsweise solche aus der Gruppe der Acrylharze aufgebaut sein. Dabei sind insbesondere Mono- und Copolymere polymerisierbarer Monomere zu nennen wie z.B. (Meth)acrylsäuremethylester, (Meth)acrylsäureethylester, (Meth)acrylsäurepropylester, (Meth)acrylsäurebutylester, Styrol und Styrolderivate, ferner Copolymere zwischen carboxylgruppentragenden polymerisierbaren Monomeren wie (Meth)acrylsäure, Itaconsäure, Maleinsäure, Maleinsäureanhydrid, Maleinsäuremonoalkylester, insbesondere mit Alkyl von 1 bis 12 C-Atomen, und polymerisierbare Monomere wie (Meth)acrylsäure, Styrol und Styrolderivate, wie z.B. α-Methylstyrol, m- oder p-Methoxystyrol, p-Hydroxystyrol. Als Beispiele seien genannt Umsetzungsprodukte von carboxylgruppenhaltigen polymeren Verbindungen mit Verbindungen, die jeweils einen Oxiranring und eine ethylenisch ungesättigte Verbindung enthalten wie z.B. Glycidyl(meth)acrylat, Acrylglycidylether und Itaconsäuremonoalkylglycidylether usw., ferner Umsetzungsprodukte von carboxylgruppenhaltigen polymeren Verbindungen mit Verbindungen, die jeweils eine Hydroxylgruppe und eine ethylenisch ungesättigte Verbindung (ungesättigte Alkohole) enthalten wie Allylalkohol, 2-Buten-4-ol, Oleylalkohol, 2-Hydroxyethyl(meth)acrylat, N-Methylolacrylamid usw.; weiterhin können derartige Bindemittelharze auch ungesättigte Verbindungen, die freie Isocyanatgruppen besitzen, enthalten.

Im Allgemeinen liegt die Äquivalenz der Ungesättigtheit (Molgewicht Bindemittelharz pro ungesättigte Verbindung) der genannten Bindemittelharze bei 200 bis 3 000, insbesondere 230 bis 1 000, um sowohl eine ausreichende Photopolymerisierbarkeit und Härte des Films zu erreichen. Der Säurewert liegt im Allgemeinen bei 20 bis 300, insbesondere 40 bis 200, um eine genügende Alkali-Entwicklungsfähigkeit nach der Belichtung des Films zu erzielen.

Das mittlere Molgewicht der einzusetzenden Bindemittelharze liegt zwischen 1 500 und 200 000, insbesondere 10 000 bis 50 000 g/mol.

Die bei der erfindungsgemäßen Verwendung der Pigmentpräparationen für Farbfilter eingesetzten organischen Lösungsmittel sind z.B. Ketone, Alkylenglykolether, Alkohole und aromatische Verbindungen. Beispiele aus der Gruppe der Ketone sind: Aceton, Methylethylketon, Cyclohexanon etc.; aus der Gruppe der Alkylenglykol-ether: Methylcellosolve (Ethylenglykolmonomethylester), Butylcellosolve (Etylen-glykolmonobutylether) Methylcellosolveacetat, Ethylcellosolveacetat, Butylcellosolveacetat, Ethylenglykolmonopropylether, Ethylenglykolmonohexylether, Ethylenglykoldimethylether, Diethylenglykolethylether, Diethylenglykoldiethylether, Propylenglykolmonomethylether, Propylenglykolmonoethylether, Propylenglykol-monopropylether, Propylenglykolmonobutylether, Propylenglykolmonomethyl-etheracetat, Diethylenglykolmethyletheracetat, Diethylenglykolethyletheracetat, Diethylenglykolpropyletheracetat, Diethylenglykolisopropyletheracetat, Diethylen-glykolbutyletheracetat, D iethylenglykol-t-butyletheracetat, Triethylenglykolmethyletheracetat, Triethylenglykolethyletheracetat, Triethylenglykolpropyletheracetat, Triethylenglykolisopropyletheracetat, Triethylenglykolbutyletheracetat, Triethylen-glykol-t-butyletheracetat, etc.; aus der Gruppe der Alkohole: Methylalkohol, Ethylalkohol, Isopropylalkohol, n-Butylalkohol, 3-Methyl-3-methoxybutanol, etc.; aus der Gruppe der aromatischen Lösungsmittel Benzol, Toluol, Xylol, N-Methyl-2-Pyrrolidon, N-Hydroxymethyl-2-Essigsäureethylester, etc.

Weitere andere Lösungsmittel sind beispielsweise 1,2-Propandioldiacetat, 3-Methyl-3-methoxybutylacetat, Essigsäureethylester, Tetrahydrofuran, etc. Die Lösungsmittel können einzeln oder in Gemischen untereinander eingesetzt werden.

Die Erfindung betrifft weiterhin einen Photolack, enthaltend wenigstens ein Pigment im obigen Sinne oder wenigstens eine erfindungsgemäße Pigmentpräparation und wenigstens ein photohärtbares Monomer sowie wenigstens einen Photoinitiator.

Die photohärtbaren Monomere enthalten im Molekül wenigstens eine reaktive Doppelbindung und gegebenenfalls andere reaktive Gruppen.

Als photohärtbare Monomere seien in diesem Zusammenhang insbesondere reaktive Lösungsmittel bzw. sog. Reaktivverdünner verstanden z.B. aus der Gruppe der mono-, di-, tri- und multifunktionelle Acrylate und Methacrylate, Vinylether, sowie Glycidylether. Als zusätzlich enthaltene reaktive Gruppen kommen in Frage Allyl-, Hydroxy-, Phosphat-, Urethan-, sek. Amin- und N-Alkoxymethylgruppen. Derartige Monomere sind dem Fachmann bekannt und beispielsweise in [Römpp Lexikon, Lacke und Druckfarben, Dr. Ulrich Zorll, Thieme Verlag Stuttgart-New York, 1998, S. 491/492] oder online unter http://www.roempp.com unter dem Stichwort 'Reaktivverdünner' aufgeführt.

Die Auswahl der Monomere richtet sich insbesondere nach der Art und Intensität der verwendeten Strahlenart der Belichtung, der gewünschten Reaktion mit dem Photoinitiator und den Filmeigenschaften. Es können auch Kombinationen von Monomeren eingesetzt werden.

Als Photoreaktionsstarter oder Photoinitiatoren seien Verbindungen verstanden, die infolge der Adsorption sichtbarer oder ultravioletter Strahlung reaktive Zwischenprodukte bilden, die eine Polymerisationsreaktion beispielsweise der oben genannten Monomeren und/oder Bindemittelharze auslösen können. Photoreaktionsstarter sind ebenfalls allgemein bekannt und können ebenfalls aus [Römpp Lexikon, Lacke und Druckfarben, Dr. Ulrich Zorll, Thieme Verlag Stuttgart-New York, 1998, S. 445/446] oder online unter http://www.roempp.com unter dem Stichwort 'Photoinitiatoren' entnommen werden.

Erfindungsgemäß besteht keine Einschränkung hinsichtlich der einzusetzenden photohärtbaren Monomeren oder Photoinitiatoren.

Die Erfindung betrifft bevorzugt Photolacke enthaltend
A) wenigstens ein "Pigment" im obigen Sinne, insbesondere in Mischung mit anderen Pigmenten oder eine darauf basierende erfindungsgemäße Pigmentpräparation,
B1) wenigstens ein photohärtbares Monomer,
B2) wenigstens einen Photoinitiator,
C1) gegebenenfalls ein organisches Lösungsmittel,
D) gegebenenfalls ein Dispergiermittel,
E) gegebenenfalls ein Bindemittelharz,
   sowie gegebenenfalls weitere Zusätze.

Erfindungsgemäß besteht auch keine Einschränkung hinsichtlich der Technologie zur Erzeugung der gefärbten Bildelementmuster auf Basis der erfindungsgemäß zu verwendenden Pigmente oder feste Pigmentpräparationen. Neben dem oben beschriebenen photolithographischen Verfahren sind andere Verfahren wie Offset-Druck, chemisches Ätzen oder Ink Jet Druck ebenso geeignet. Die Auswahl der geeigneten Bindemittelharze und Lösungsmittel bzw. Pigment-Trägermedien sowie weitere Zusätze sind auf das jeweilige Verfahren abzustimmen. Beim Ink Jet Verfahren, worunter sowohl der thermische als auch mechanische und piezzo-mechanische Ink Jet Druck verstanden werden, kommen neben rein organischen auch wässrig-organische Trägermedien für die Pigmente und gegebenenfalls Bindemittelharze in Frage, wässrig-organische Trägermedien werden sogar bevorzugt, wobei als organische Lösungsmittel, die obengenannten in Frage kommen.

Die Erfindung betrifft weiterhin Verbindungen der Formel (IVa) 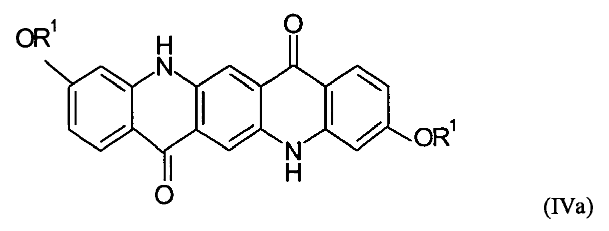 worin R¹ ein fluor-haltiger aliphatischer Rest, insbesondere ein fluor-haltiger C₁-C₈-Rest bedeutet, vorzugsweise für Fluormethyl, Trifluormethyl, 2-Fluorethyl, 2,2,2-Trifluorethyl, Pentafluorethyl, 2,2,3,3-Tetrafluorpropyl, Perfluorbutyl oder Perfluoroctyl steht.

Die Erfindung betrifft weiterhin ein Verfahren zur Herstellung von Verbindungen der Formel (IVa) **dadurch gekennzeichnet, dass** man ein, vorzugsweise in 3 Stellung, OR¹ substituiertes Anilin, insbesondere ein Anilin der Formel (IVb) 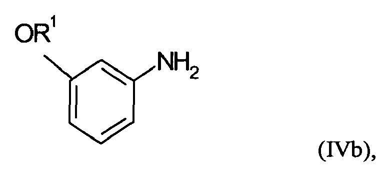 worin R¹ ein fluor-haltiger aliphatischer Rest, insbesondere ein fluor-haltiger C₁-C₈-Rest bedeutet, vorzugsweise für Fluormethyl, Trifluormethyl, 2-Fluorethyl, 2,2,2-Trifluorethyl, Pentafluorethyl, 2,2,3,3-Tetrafluorpropyl, Perfluorbutyl oder Perfluoroctyl steht, mit Dimethylsuccinylsuccinat, vorzugsweise in Gegenwart eines Lösungsmittels und sauren Katalysators, umsetzt, dann oxidiert und verseift und anschliessend die so erhaltene Dicarbonsäure, vorzugsweise nach Isolierung und Trocknung, in Schwefelsäure oder Polyphosphorsäure zum Chinacridon kondensiert.

Die Erfindung betrifft weiterhin Verbindungen der Formel (Va) 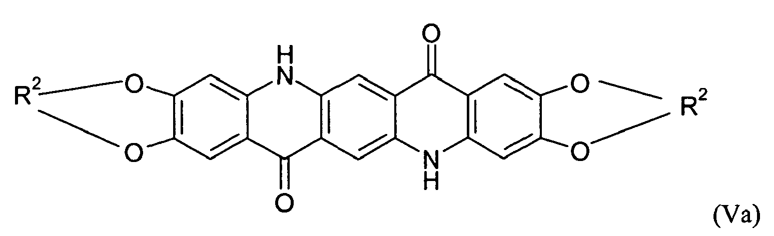 worin
R² eine fluor-substituierte C₁-C₃-Alkylen Einheit bedeutet, insbesondere für - CF₂ -, -CF₂-CF₂-,

-CHF-CHF- oder - CF₂-CF₂-CF₂- steht.

Die Erfindung betrifft weiterhin ein Verfahren zur Herstellung von Verbindungen der Formel (Va), **dadurch gekennzeichnet, dass** man ein vorzugsweise in 3,4-Stellung, -O-R²-O- anelliertes Anilin, insbesondere ein Anilin der Formel (Vb) 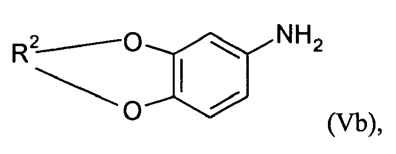 worin R² eine fluor-substituierte C₁-C₃-Alkylen Einheit bedeutet, insbesondere für - CF₂ -, -CF₂-CF₂-, -CHF-CHF- oder - CF₂-CF₂-CF₂- steht, mit Dimethylsuccinylsuccinat, vorzugsweise in Gegenwart eines Lösungsmittels und sauren Katalysators, umsetzt, dann oxidiert und verseift und anschliessend die so erhaltene Dicarbonsäure vorzugsweise nach Isolierung und Trocknung in Schwefelsäure oder Polyphosphorsäure zum Chinacridon kondensiert.

### Beispiele:

### Beispiel 1

### a) Zwischenprodukt

1000g Methanol wurden vorgelegt. 1,00 mol p-Trifluormethoxyanilin wurden zugegeben. 0,44 mol DMSS (Dimethylsuccinylsuccinat) und 6g Schwefelsäure 98% wurden eingetragen. 2 Stunden wurde unter Druck bei 85°C umgesetzt. Anschliessend wurden bei Raumtemperatur 0,44 mol m-Nitrobenzolsulfonsäure-Na-salz eingetragen. Danach wurden 2,05 mol Kaliumhydroxidlösung 50% zugetropft und 37g Wasser. 3h wurde unter Druck bei 85°C umgesetzt.

Weiter wurde mit 1500g Wasser verdünnt, mit Phosphorsäure (85%ig) auf pH 4 eingestellt und 1 Stunde nachgerührt, isoliert, nachgewaschen und getrocknet.

Ausbeute: 95% Dicarbonsäure der Formel

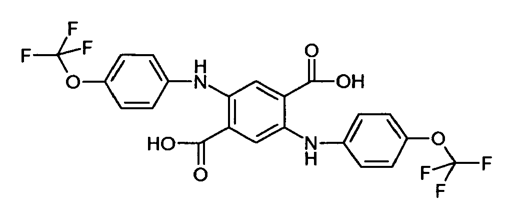

### b) Cyclisierung

450g Polyphosphorsäure (Gehalt: 84% P₂O₅) wurden vorgelegt und auf ca. 100 °C erwärmt. 0,13 mol Dicarbonsäure hergestellt in Beispiel 1 wurden im Laufe von 30 min eingetragen. 2 Stunden wurde bei 105°C nachgerührt. In 2,5 Stunden wurden 375 g Phosphorsäure 75% bei 105°C zugetropft.

Die viskose Suspension wurde im Laufe von 30 min in eine Vorlage von 500g Wasser getropft und 30 min nachgerührt. Das Produkt wurde isoliert, salzfrei gewaschen, anschliessend in 1000g Kaliumtriphosphatlösung 2% angeschlämmt, erneut isoliert und neutral gewaschen. Der Presskuchen wurde mit Methanol angeschlagen und anschliessend mit Wasser methanolfrei gewaschen und getrocknet.

Ausbeute: 91% Trifluormethoxychinacridon (Formel II) Kristallmodifikation A 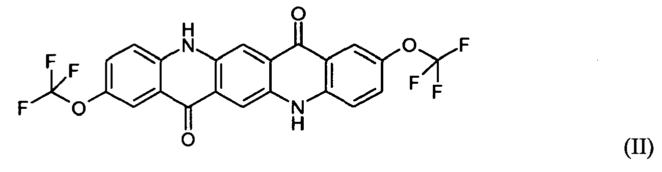 BET: 14 m²/g

### c) Herstellung der PVC-Probe zur Bestimmung des Farbtons und der Dispergierhärte

Zur Bestimmung der Dispergierhärte wurden 100 g PVC-Paste , hergestellt aus 4,2 Teilen Vestolit ® E 7004 (Emulsions-PVC-Pulver), 1,8 Teilen Diisooctylphthalat, 0,15 Teilen Stabilisator Baerostab® UBZ 770 (flüssiger Barium-Zink-Stabilisator) und 0,125 Teilen Moltopren® Weißpaste RUN 01 (Pigmentpräparation enthaltend 50% TiO₂) bei 150°C auf ein Labor-Mischwalzwerk der Fa. Collin aufgebracht. Der Walzenspalt betrug 0,8 mm.

0,1 g der Probe wurden auf das PVC-Fell aufgebracht und der Walzenpalt auf 0,12mm eingestellt. Das Walzenfell wurde abgenommen und wieder aufgegeben. Dieser Vorgang wurde achtmal wiederholt. Der Walzenspalt wurde auf 0,8 mm eingestellt und das Walzenfell abgenommen. Aus dem Walzenfell wurde ein Prüfmuster von 60 X 60 mm ausgestanzt.

Der Rest des Felles wurde nun auf das Walzwerk bei 25°C gegeben. Der Walzenspalt betrug 0,2 mm. Das Walzenfell wurde abgenommen und wieder aufgegeben. Dieser Vorgang wurde 15 mal wiederholt. Das vom Kaltwalzen nicht mehr glatte Fell wurde auf die Walze bei 150 °C und einem Walzenspalt von 0,8 mm gegeben. Nach 60 s wurde das Fell abgenommen und ein Prüfmuster von 60 X 60 mm ausgestanzt. Die Umdrehungszahl der Walze wurde immer konstant auf 20 upm und die Friktion auf 1:1,1 gehalten.

Die Dispergierhärte ist die prozentuale Zunahme der Farbstärke nach Walzen bei 25 °C.

### Dispergierhärte: 78

Die Herstellung der transparenten Prüfmuster erfolgte in gleicher Weise, allerdings unter der Verwendung einer Prüfpaste, bestehend aus 4,2 Teilen Vestolit® E 7004 (E-PVC-Pulver), 1,8 Teilen Diisooctylphthalat und 0,15 Teilen Stabilisator Baerostab® UBZ 770 (flüssiger Barium-Zink-Stabilisator).

Die Farbtöne wurden mit einem Spektrometer der Fa. Gretag Macbeth bestimmt. Dabei wurden die Proben mit 10° Beobachterwinkel, Lichtart D65 und ohne Glanzfalle gemessen. Remissionsmessung:

| | | | | |
|---|---|---|---|---|
| L*: 65,82 | a*: 38,34 | b*: -14,39 | C*: 40,95 | h°: 339,44 |

### Beispiel 2

110 g Schwefelsäure 98% wurden bei 20°C vorgelegt. 14,9 g Trifluormethoxy-chinacridon (hergestellt in Beispiel 1) wurden im Laufe von 30 min bei 10 - 25°C eingetragen. Die Lösung wurde mit 20 g Schwefelsäure verdünnt und innerhalb von 20 min in 500 ml Methanol bei ca. 20°C getropft. Die Suspension wurde mit 800 g Wasser verdünnt, isoliert, neutral gewaschen und getrocknet.

Ausbeute: 13g Trifluormethoxychinacridon (Formel II) Kristallmodifikation B
BET: 46 m²/g
Herstellung der PVC-Probe zur Bestimmung des Farbtons und der Dispergierhärte anolog zu Beispiel 1c

Dispergierhärte: 600

| | | | | |
|---|---|---|---|---|
| L*: 63,37 | a*: 43,64 | b*: -17,11 | C*: 46,87 | h°: 338,59 |

### Beispiel 3

370g Polyphosphorsäure (Gehalt: 84% P₂O₅) wurden vorgelegt und auf ca. 100 °C erwärmt. 37,2 g Dicarbonsäure hergestellt in Beispiel 1a) wurden im Laufe von 30 min eingetragen. 1 Stunde wurde bei 105°C nachgerührt. Die viskose Lösung wurde auf 500 ml Methanol von 60°C getropft. 1 Stunde wurde bei 60°C nachgerührt, isoliert, mit Wasser gewaschen und getrocknet.
Ausbeute: 31,6 g Trifluormethoxychinacridon (Formel II) Kristallmodifikation B
BET: 47 m²/g
Herstellung der PVC-Probe zur Bestimmung des Farbtons und der Dispergierhärte anolog zu Beispiel 1c

Dispergierhärte: 106

| | | | | |
|---|---|---|---|---|
| L*: 64,58 | a*: 43,29 | b*: -14,82 | C*: 45,76 | h°: 341,11 |

### Beispiel 4

1200g Schwefelsäure 98%ig wurden vorgelegt. 150 g Trifluormethoxychinacridon (hergestellt in Beispiel 1) wurden im Laufe von 30 min bei 10 - 25°C eingetragen und über eine Glasfritte geklärt.

1000 ml Wasser wurden vorgelegt, 1500g Eis wurden eingetragen. Im Laufe von 30 Minuten wurde die Schwefelsäurelösung zugetropft, wobei die Temperatur durch Zugabe von ca. 2500 g Eis auf ca. - 5°C gehalten wurde. Es wurde ca. 30 Minuten nachgerührt, isoliert und salzfrei gewaschen.
Ausbeute: 146 g Trifluormethoxychinacridon (Formel II) Kristallmodifikation B
BET: 72 m²/g

### c) Herstellung der PVC-Probe zur Bestimmung des Farbtons und der Dispergierhärte analog zu Beispiel 1c

Dispergierhärte: 20

| | | | | |
|---|---|---|---|---|
| L*: 66,57 | a*: 43,40 | b*: -12,91 | C*: 45,28 | h°: 343,44 |

### Beispiel 5

Verbindung (IV) wurde (ausgehend von m-Trifluormethoxyanilin) analog zum Beispiel 1 hergestellt und geprüft.

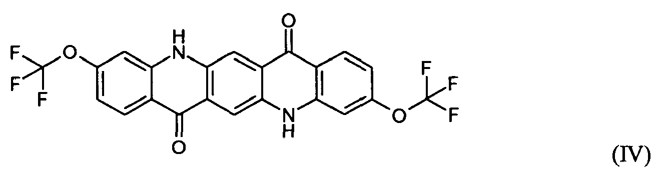

### BET: 46 m²/g

Dispergierhärte: 100

| | | | | |
|---|---|---|---|---|
| L*: 64,85 | a*: 30,28 | b*: -1,16 | C*: 30,30 | h°: 357,81 |

### Verwendungsbeispiele

Herstellung einer roten Zubereitung und Verwendung zur Herstellung eines roten Farbfilters
**Verwendungsbeispiel 1** (nicht erfindungsgemäß):
Einsatzpigment: Pigment Rot 122

In einem Rührbehälter wurden 774 Gew.-Teile Methoxybutylacetat und 286 Gew.-Teile einer 21%igen Lösung eines alkalisch löslichen Copolymeren (Bindemittelharz) auf Basis von Benzylmethacrylat (70T) / 2-Hydroxyethylmethacrylat (15T) / Methacrylsäure (15T), Molgewicht ca. 25.000 g/mol, in Methoxypropylacetat homogen gemischt.

Anschließend wurden 100 Gew.Teile Pigment Rot 122, das zuvor bei 70°C auf eine Restfeuchte von weniger als 1 Gew% getrocknet wurde, homogen eingetragen.

Die Pigmentsuspension wurde in einer horizontalen, geschlossenen Perlmühle unter Einsatz von Yttrium-stabilisierten Zirkonoxid-Perlen (Durchmesser 0,6 bis 1,0 mm) in mehreren Passagen gemahlen, bis ein effektiver Partikeldurchmesser (gemessen mit Laserstreulichtkorrelationsspektroskopie an einer ca. 0,5 Gew.-% Verdünnung in Methoxypropylacetat) von weniger als 150 nm verbunden mit einer Polydispersität von weniger als 0,14 erreicht war. (Vergleichsweise konnte elektronenmikroskopisch an einem getrockneten Film aus einer 1 %igen Verdünnung in Methoxypropylacetat eine sehr enge Partikelgrößenverteilung mit 95% Anzahl der Teilchen unter 100 nm festgestellt werden.)

### Herstellung eines Photolacks

Zu 1000 Gew.-Teilen der so erhaltenen Zubereitung wurden 34,5 Gew.-Teile Trimethylolpropantriacrylat (monomerer Reaktivverdünner) und 13,8 Gew.Teile eines Photoreaktionsstarters auf Basis von Benzophenon und N,N'-Tetraethyl-4,4'-diaminobenzophenon im Verhältnis von 3/1 Gew.Teilen unter Rühren homogen eingetragen.

Man erhielt einen UV-strahlungshärtbaren Photolack, der auf einem transparenten Substrat aufgetragen und zum Farbfilter entwickelt wurde.

Hierzu wurde der Photolack mittels Spin-Coating auf ein 300 x 350 mm großes Stück gereinigtes Borosilikatglas (Corning® 7059, Owens Corning Corp.) beschichtet und bei 110°C 5 Minuten in einem Ofen unter Reinbedingungen zu einem ca. 1,5 - 2µm dicken Film getrocknet.

Anschließend wurde der Film nach Abkühlung mittels einer Negativmaske zur Erzielung des gewünschten Streifenbildmusters und einer Ultrahochdruck-Quecksilberdampflampe bei einer Dosis von 200 mJ/cm² UV-belichtet und dann mittels 0,06 %iger wässriger Kaliumhydroxidlösung bei Raumtemperatur entwickelt, mit vollentsalztem Wasser gereinigt und getrocknet. Anschließend erfolgte eine 30 minütige Nachhärtung bei 235°C in einem Ofen unter Reinbedingungen.

**Verwendungsbeispiel 2** (erfindungsgemäß):
Einsatzpigment: Pigment aus Beispiel 3

In einem Rührbehälter wurden 774 Gew.-Teile Methoxybutylacetat und 286 Gew.-Teile einer 21%igen Lösung eines alkalisch löslichen Copolymeren (Bindemittelharz) auf Basis von Benzylmethacrylat (70T) / 2-Hydroxyethylmethacrylat (15T) / Methacrylsäure (15T), Molgewicht ca. 25.000 g/mol, in Methoxypropylacetat homogen gemischt.

Anschließend wurden 100 Gew.Teile Pigment aus Beispiel 3, das zuvor bei 70°C auf eine Restfeuchte von weniger als 1 Gew% getrocknet wurde, homogen eingetragen.

Die Pigmentsuspension wurde in einer horizontalen, geschlossenen Perlmühle unter Einsatz von Yttrium-stabilisierten Zirkonoxid-Perlen (Durchmesser 0,6 bis 1,0 mm) in mehreren Passagen gemahlen, bis ein effektiver Partikeldurchmesser (gemessen mit Laserstreulichtkorrelationsspektroskopie an einer ca. 0,5 Gew.-% Verdünnung in Methoxypropylacetat) von weniger als 150 nm verbunden mit einer Polydispersität von weniger als 0,14 erreicht war. (Vergleichsweise konnte elektronenmikroskopisch an einem getrockneten Film aus einer 1%igen Verdünnung in Methoxypropylacetat eine sehr enge Partikelgrößenverteilung mit 95% Anzahl der Teilchen unter 100 nm festgestellt werden.)

### Herstellung eines Photolacks

Zu 1000 Gew.-Teilen der so erhaltenen Zubereitung wurden 34,5 Gew.-Teile Trimethylolpropantriacrylat (monomerer Reaktivverdünner) und 13,8 Gew.Teile eines Photoreaktionsstarters auf Basis von Benzophenon und N,N'-Tetraethyl-4,4'-diaminobenzophenon im Verhältnis von 3/1 Gew.Teilen unter Rühren homogen eingetragen.

Man erhielt einen UV-strahlungshärtbaren Photolack, der auf einem transparenten Substrat aufgetragen und zum Farbfilter entwickelt wurde.

Hierzu wurde der Photolack mittels Spin-Coating auf ein 300 x 350 mm großes Stück gereinigtes Borosilikatglas (Corning® 7059, Owens Corning Corp.) beschichtet und bei 110°C 5 Minuten in einem Ofen unter Reinbedingungen zu einem ca. 1,5 - 2µm dicken Film getrocknet.

Anschließend wurde der Film nach Abkühlung mittels einer Negativmaske zur Erzielung des gewünschten Streifenbildmusters und einer Ultrahochdruck-Quecksilberdampflampe bei einer Dosis von 200 mJ/cm² UV-belichtet und dann mittels 0,06 %iger wässriger Kaliumhydroxidlösung bei Raumtemperatur entwickelt, mit vollentsalztem Wasser gereinigt und getrocknet. Anschließend erfolgte eine 30 minütige Nachhärtung bei 235°C in einem Ofen unter Reinbedingungen.

Der so erhaltene rote erfindungsgemäße Farbfilter 2, hergestellt gemäß dem Verwendungsbeispiel 2, ausgehend von dem Beispiel 3, besaß gegenüber dem nicht-erfindungsgemäßen Farbfilter 1, hergestellt gemäß dem Verwendungsbeispiel 1, ausgehend von Pigment Rot 122, eine deutlich verbesserte spektrale Transparenz. Die Farbreinheit und Brillanz des Farbfilters 2 ist ausgezeichnet.

### Verwendungsbeispiel 3 (erfindungsgemäß):

Einsatzpigment: 80% Pigment aus Beispiel 3
20% mit Melamin interkalliertes 1:1 1 Nickelazobarbitursäurepigment
Herstellung einer roten Zubereitung und Verwendung zur Herstellung eines roten Farbfilters

Mit dem gleichen Verfahren wie in Verwendungsbeispiel 2 beschrieben jedoch unter Einsatz von 80 Gew.Teilen des Pigmentes gemäß Beispiel 3 und 20 Gew.Teilen eines mit Melamin interkallierten Nickelazobarbitursäurekomplexes (hergestellt nach Beispiel 2 aus DE 10 2005 033 581 B4) wurde eine Zubereitung hergestellt, die zur Herstellung roter Photolacke für Farbfilter sehr gut geeignet ist.

Ein wie in Verwendungsbeispiel 2 beschrieben hergestellter Photolack und damit hergestellter roter erfindungsgemäßer Farbfilter besaß sehr gute spektrale Transparenzeigenschaften sowie ausgezeichnete Farbreinheit und Brillanz.

### Verwendungsbeispiel 4 (erfindungsgemäß):

Einsatzpigment: 40% Pigment aus Beispiel 3
40% C.I. Pigment Rot 254
20% mit Melamin interkalliertes 1:1 1 Nickelazobarbitursäurepigment
Herstellung einer roten Zubereitung und Verwendung zur Herstellung eines roten Farbfilters

Mit dem gleichen Verfahren wie in Verwendungsbeispiel 2 beschrieben jedoch unter Einsatz von 40 Gew.Teilen des Pigmentes gemäß Beispiel 3, 40 Gew.Teilen C.I. Pigment Rot 254 und 20 Gew.Teilen eines mit Melamin interkallierten Nickelazobarbitursäurekomplexes (hergestellt Beispiel 2 in DE 10 2005 033 581 B4) wurde eine Zubereitung hergestellt, die zur Herstellung roter Photolacke für Farbfilter sehr gut geeignet ist.

Ein wie in Verwendungsbeispiel 2 beschrieben hergestellter Photolack und damit hergestellter roter erfindungsgemäßer Farbfilter besaß sehr gute spektrale Transparenzeigenschaften sowie ausgezeichnete Farbreinheit und Brillanz.

### Verwendungsbeispiel 5 (erfindungsgemäß):

Einsatzpigment: Pigment aus Beispiel 4

Herstellung einer roten Zubereitung und Verwendung zur Herstellung eines roten Farbfilters

Mit dem gleichen Verfahren wie in Verwendungsbeispiel 2 beschrieben jedoch unter Einsatz des Pigmentes gemäß Beispiel 4 wurde eine Zubereitung hergestellt, die zur Herstellung roter Photolacke für Farbfilter sehr gut geeignet ist.

Ein wie in Verwendungsbeispiel 2 beschrieben hergestellter Photolack und damit hergestellter roter erfindungsgemäßer Farbfilter besaß sehr gute spektrale Transparenzeigenschaften sowie ausgezeichnete Farbreinheit und Brillanz.

### Verwendungsbeispiel 6 (erfindungsgemäß):

Einsatzpigment: Pigment aus Beispiel 5

Herstellung einer roten Zubereitung und Verwendung zur Herstellung eines roten Farbfilters

Mit dem gleichen Verfahren wie in Verwendungsbeispiel 2 beschrieben jedoch unter Einsatz des Pigmentes gemäß Beispiel 5 wurde eine Zubereitung hergestellt, die zur Herstellung roter Photolacke für Farbfilter sehr gut geeignet ist.

Ein wie in Verwendungsbeispiel 2 beschrieben hergestellter Photolack und damit hergestellter roter erfindungsgemäßer Farbfilter besaß sehr gute spektrale Transparenzeigenschaften sowie ausgezeichnete Farbreinheit und Brillanz.

### Verwendungsbeispiel 7 (erfindungsgemäß):

Einsatzpigment: Pigment der Formel (III)

Herstellung einer roten Zubereitung und Verwendung zur Herstellung eines roten Farbfilters

Mit dem gleichen Verfahren wie in Verwendungsbeispiel 2 beschrieben jedoch unter Einsatz eines Pigmentes der Formel (III) wurde eine Zubereitung hergestellt, die zur Herstellung roter Photolacke für Farbfilter sehr gut geeignet ist.

Ein wie in Verwendungsbeispiel 2 beschrieben hergestellter Photolack und damit hergestellter roter erfindungsgemäßer Farbfilter besaß sehr gute spektrale Transparenzeigenschaften sowie ausgezeichnete Farbreinheit und Brillanz.

## Patentansprüche

1. Verwendung von Chinacridonen der Formel (I) 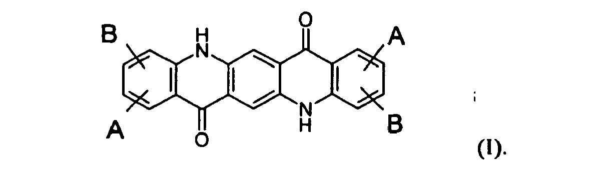 worin
A für einen durch ein oder mehrere Fluoratome substituierten organischen Rest steht,
B für H, F, Cl, Br oder für einen ggf. substituierten organischen Rest steht, der ggf. zusammen mit A einen Ring bilden kann
in Farbfiltern für LCD.

2. Verwendung nach Anspruch 1, worin
A für C₁-C₈-Alkyl, C₁-C₈-Alkoxy, Phenyl oder Phenoxy steht, das jeweils durch ein oder mehrere Fluoratome substituiert ist,
und
B für H steht,
oder
A und B gemeinsam für eine durch ein oder mehrere Fluor-Atome substituierte Brücke, die mit zwei benachbarten C-Atomen des Benzorings der Formel (I) einen fünf-, sechs- oder siebengliedrigen Ring bildet, der carbocyclisch ist oder Heteroatome wie O, S oder N enthalten kann, stehen.

3. Verwendung nach Anspruch 1, worin
A für Fluormethyl, Trifluormethyl, 2-Fluorethyl, 2,2,2-Trifluorethyl, Pentafluorethyl, 2,2,3,3-Tetrafluorpropyl, Perfluorbutyl, Perfluoroctyl oder die entsprechenden Alkoxyreste, insbesondere für Trifluormethyl oder Trifluormethoxy steht und
B für H steht,
oder A und B gemeinsam für -OCF₂O-, -OCF₂CF₂O-, -OCHFCHFO- oder - OCF₂CF₂CF₂O- steht.

4. Verwendung nach Anspruch 1, **dadurch gekennzeichnet, dass** als Pigment der Formel **(I)** wenigstens ein Pigment der Formel (II) bis (VIII) verwendet wird,

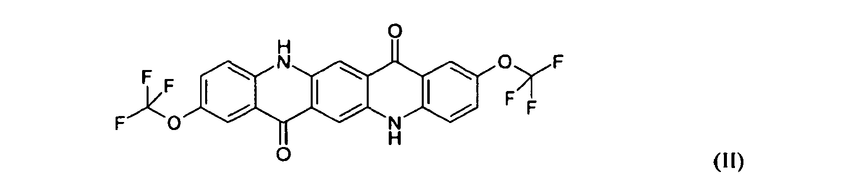

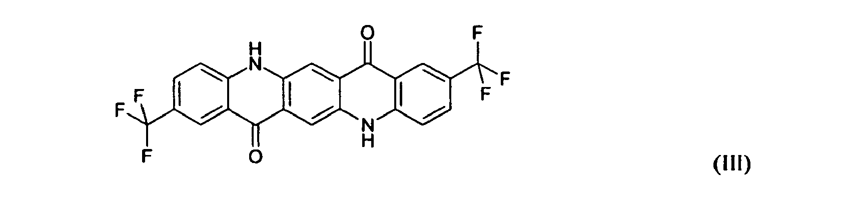

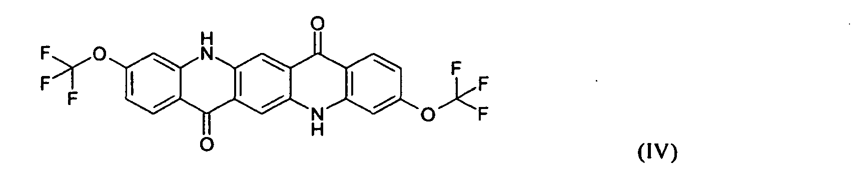

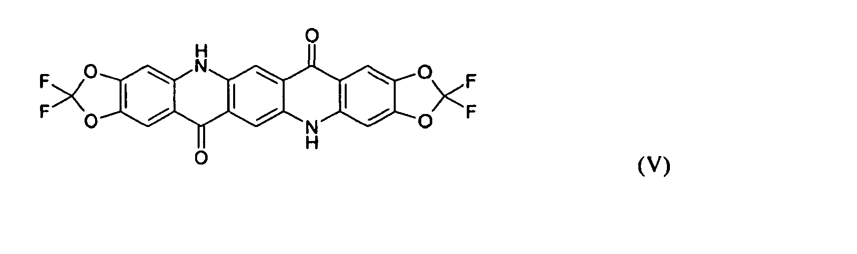

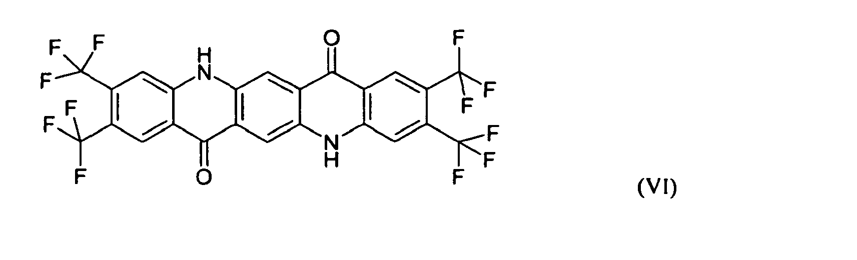

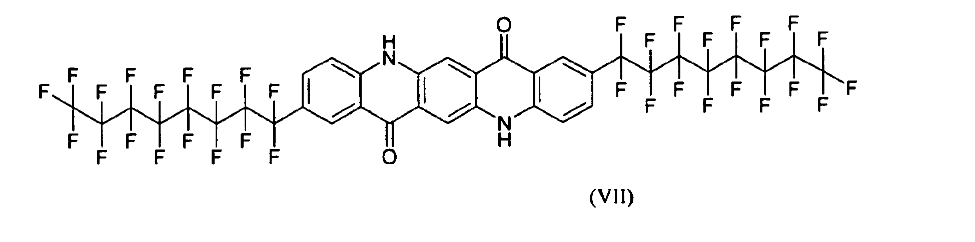

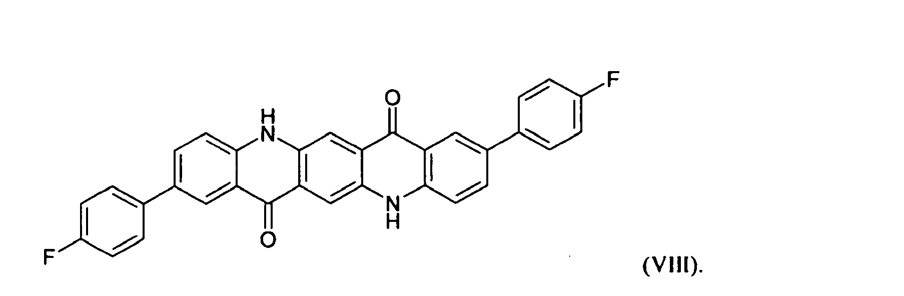

5. Verwendung nach wenigstens einem der Ansprüche 1 bis 4, **dadurch gekennzeichnet, dass** das Pigment der Formel (I) in Kombination mit anderen Pigmenten verwendet wird.

6. Verwendung nach Anspruch 1, **dadurch gekennzeichnet, dass** das Pigment der Formel (I) eine Oberfläche nach B.E.T. von 40 - 200 m²/g aufweist.

7. Verwendung nach Anspruch I, **dadurch gekennzeichnet, dass** das Pigment der Formel (I) eine Dispergierhärte von 10 bis 500 besitzt, gemessen nach DIN 53775, Teil 7, wobei die Temperatur der Kaltwalzung 25°C und die Temperatur der Warmwalzung 150°C beträgt.

8. Photolack, enthaltend wenigstens ein photohärtbares Monomer, wenigstens einen Photoinitiator und wenigstens ein Pigment der Formel (I), wie in einem der Ansprüche I bis 11 definiert.

9. Farbfilter, enthaltend wenigstens ein Pigment, wie in einem der Ansprüche 1 bis 7 definiert.

10. Flüssigkristallanzeige, enthaltend wenigstens einen Farbfilter gemäß Anspruch 9.

11. Verbindung der Formel (II) 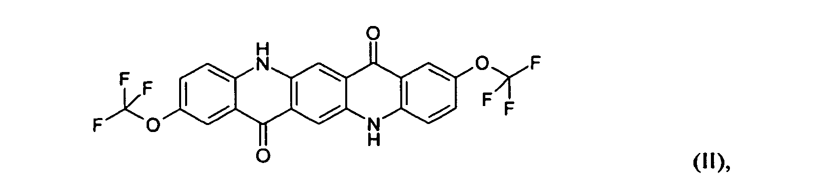 die im Röntgenbeugungs-diagramm (Cu-K_{α}-Strahlung) Linien bei folgenden d-Werten aufweist:
d: 4,22; d: 3,55; d: 3,31 (B-Modifikation)

12. Verbindung der Formel (IVa) 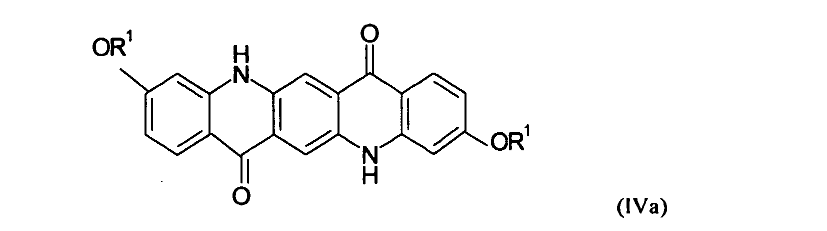 worin R¹ ein fluor-haltiger aliphatischer Rest, insbesondere ein fluor-haltiger C₁-C₈-Rest, vorzugsweise Fluormethyl, Trifluormethyl, 2-Fluorethyl, 2,2,2-Trifluorethyl, Pentafluorethyl, 2,2,3,3-Tetrafluorpropyl, Perfluorbutyl oder Perfluoroctyl ist, insbesondere eine Verbindung der Formel (IV)

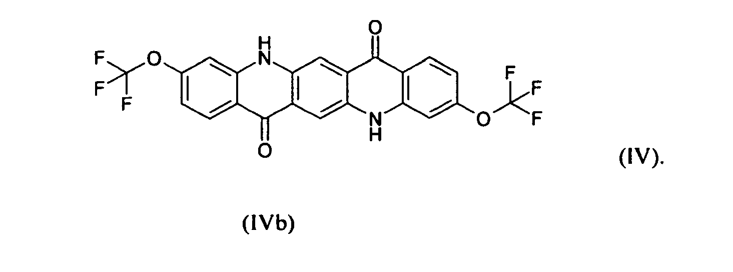

13. Verbindung der Formel (Va) 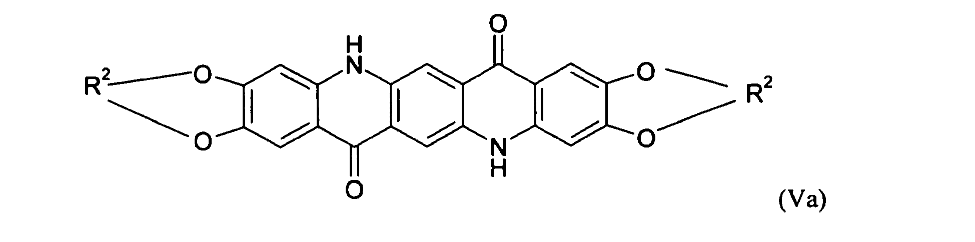 worin R² eine fluor-substituierte C₁-C₃-Alkyleneinheit bedeutet, insbesondere -CF₂-, -CF₂CF₂-, -CHFCHF- oder -CF₂CF₂CF₂-. insbesondere eine Verbindung der Formel (V)

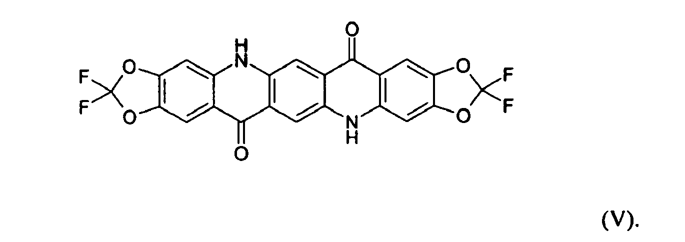

14. Mischungen, enthaltend
- wenigstens ein Pigment der Formel (I)
- wenigstens ein weiteres Rotpigment aus der Gruppe C.I. Pigment Rot 122, C.I. Pigment Rot 149, C.I. Pigment Rot 177, C.I. Pigment Rot 179, C.I. Pigment Rot 254 und C.I. Pigment Violett 19.

15. Mischungen, enthaltend
- wenigstens ein Pigment der Formel (I) und
- wenigstens ein Gelbpigment mit einer Absorptionsbande im Bereich von 400 bis 520 nm, insbesondere Gelbpigmente ausgewählt aus der Gruppe C.I. Pigment Gelb 138, C.I. Pigment Gelb 139, C.I. Pigment Gelb 150 und ein Melamin interkaliertes 1:1 Nickelazobarbitursäurekomplex-Pigment.

## Claims

1. Use of quinacridones of the formula (I) 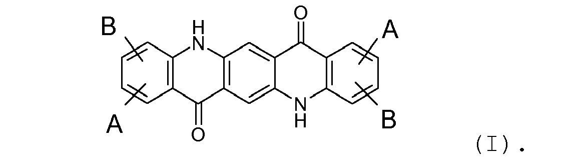 in which
A is an organic radical substituted by one or more fluorine atoms,
B is H, F, Cl, Br or an optionally substituted organic radical which may optionally together with A form a ring
in colour filters for LCDs.

2. Use according to Claim 1, in which
A is C₁-C₈-alkyl, C₁-C₈-alkoxy, phenyl or phenoxy each of which is substituted by one or more fluorine atoms, and
B is H,
or
A and B together are a bridge which is substituted by one or more fluorine atoms and which, with two adjacent C atoms of the benzo ring of the formula (I), forms a five-, six- or seven-membered ring which is carbocyclic or may contain heteroatoms such as O, S or N.

3. Use according to Claim 1, in which
A is fluoromethyl, trifluoromethyl, 2-fluoroethyl, 2,2,2-trifluoroethyl, pentafluoroethyl, 2,2,3,3-tetrafluoropropyl, perfluorobutyl, perfluorooctyl or the corresponding alkoxy radicals, especially trifluoromethyl or trifluoromethoxy, and
B is H,
or A and B together are -OCF₂O-, -OCF₂CF₂O-, - OCHFCHFO- or -OCF₂CF₂CF₂O-.

4. Use according to Claim 1, **characterized in that** at least one pigment of the formula (II) to (VIII) is used as pigment of the formula (I),

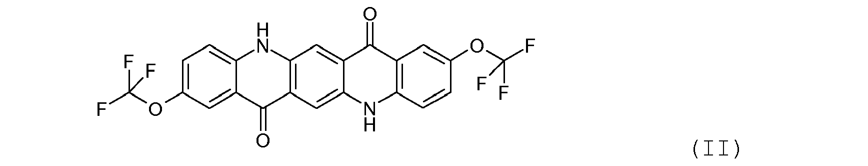

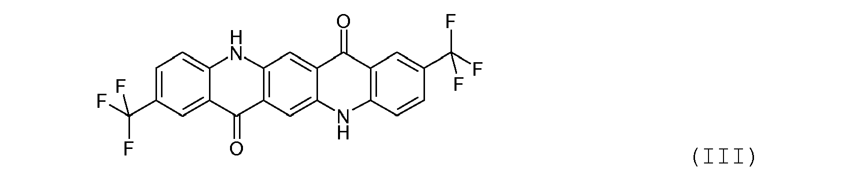

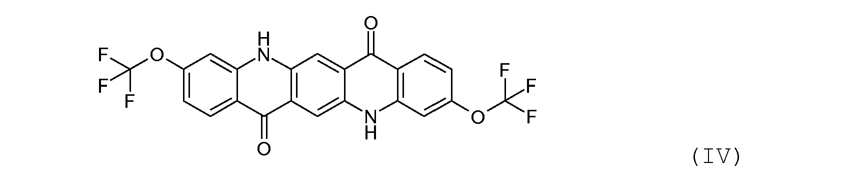

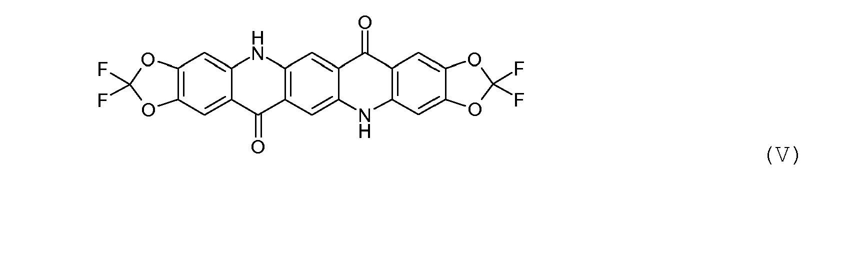

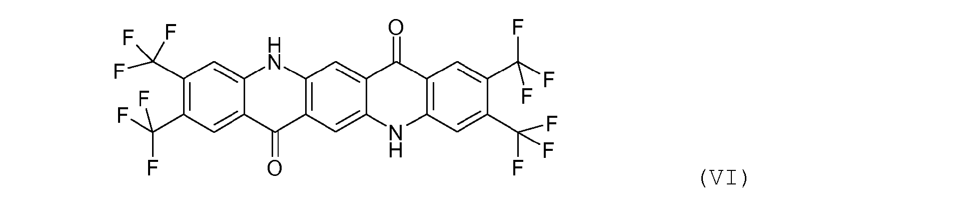

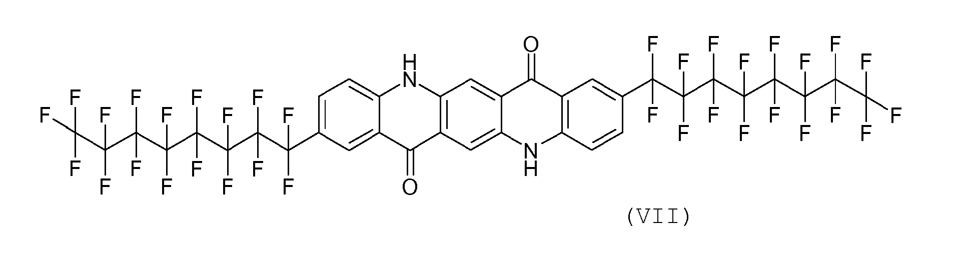

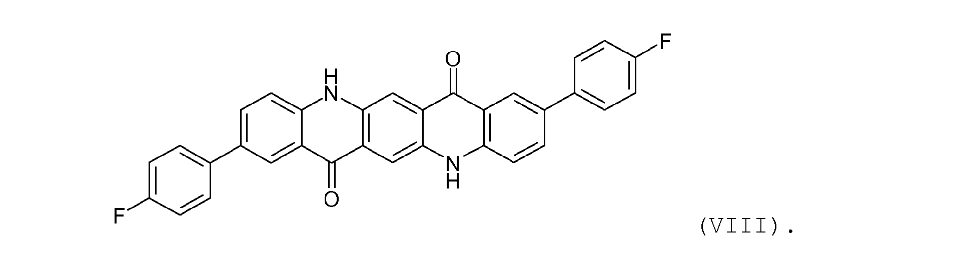

5. Use according to at least one of Claims 1 to 4, **characterized in that** the pigment of the formula (I) is used in combination with other pigments.

6. Use according to Claim 1, **characterized in that** the pigment of the formula (I) has a B.E.T. surface area of 40 - 200 m²/g.

7. Use according to Claim 1, **characterized in that** the pigment of the formula (I) possesses a dispersion harshness of 10 to 500, measured according to DIN 53775, part 7, the temperature of cold rolling being 25°C and the temperature of hot rolling being 150°C.

8. Photoresist comprising at least one photocurable monomer, at least one photoinitiator and at least one pigment of the formula (I) as defined in any of Claims 1 to 7.

9. Colour filter comprising at least one pigment as defined in any of Claims 1 to 7.

10. Liquid-crystal display comprising at least one colour filter according to Claim 10.

11. Compound of the formula (II) 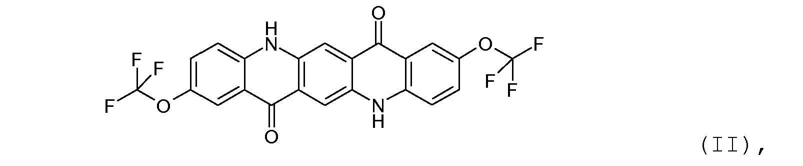 which in the X-ray diffraction diagram (Cu-K_{α} radiation) has lines at the following d values:
d: 4.22; d: 3.55; d: 3.31 (B modification).

12. Compound of the formula (IVa) 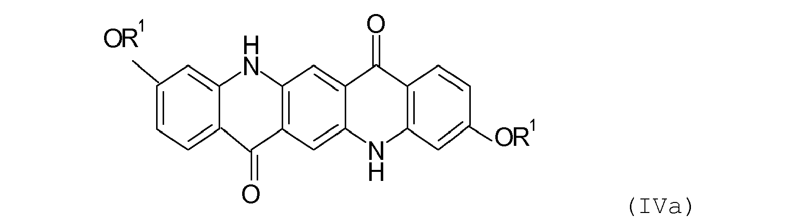 in which R¹ is a fluorine-containing aliphatic radical, especially a fluorine-containing C₁-C₈-radical, preferably fluoromethyl, trifluoromethyl, 2-fluoroethyl, 2,2,2-trifluoroethyl, pentafluoroethyl, 2,2,3,3-tetrafluoropropyl, perfluorobutyl or perfluorooctyl, especially a compound of the formula (IV)

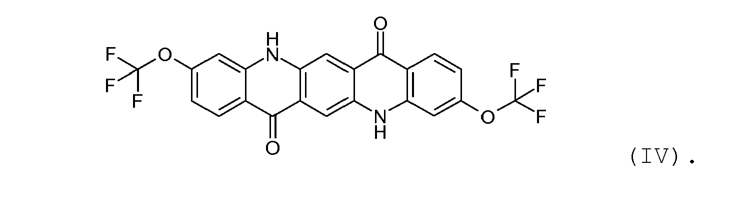

13. Compound of the formula (Va) 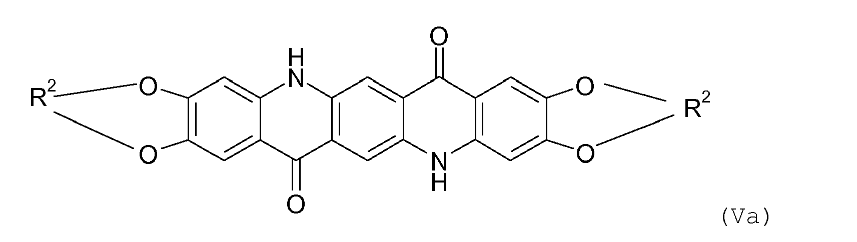 in which R² is a fluorine-substituted C₁-C₃-alkylene unit, especially -CF₂-, -CF₂CF₂-, -CHFCHF-or -CF₂CF₂CF₂-, especially a compound of the formula (V)

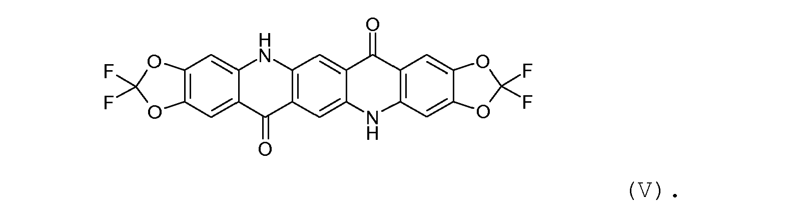

14. Mixtures comprising
- at least one pigment of the formula (I)
- at least one further red pigment from the group consisting of C.I. Pigment Red 122, C.I. Pigment Red 149, C.I. Pigment Red 177, C.I. Pigment Red 179, C.I. Pigment Red 254 and C.I. Pigment Violet 19.

15. Mixtures comprising
- at least one pigment of the formula (I) and
- at least one yellow pigment having an absorption band in the range from 400 to 520 nm, especially yellow pigments selected from the group consisting of C.I. Pigment Yellow 138, C.I. Pigment Yellow 139, C.I. Pigment Yellow 150 and a melamine-intercalated 1:1 nickel-azobarbituric acid complex pigment.

## Revendications

1. Utilisation de quinacridones de formule (I) 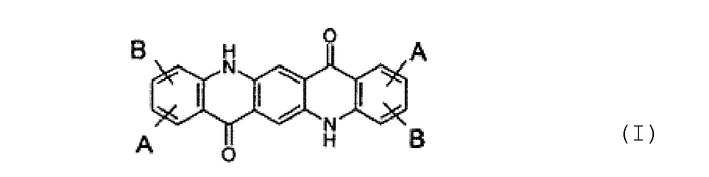 dans laquelle
A représente un radical organique substitué par un ou plusieurs atomes de fluor,
B représente H, F, Cl, Br ou un radical organique éventuellement substitué, qui éventuellement peut conjointement avec A former un cycle
dans des filtres colorés pour LCD.

2. Utilisation selon la revendication 1, dans laquelle
A représente un groupe alkyle en C₁-C₈, alcoxy en C₁-C₈, phényle ou phénoxy, qui est dans chaque cas substitué par un ou plusieurs atomes de fluor, et
B représente H,
ou
A et B ensemble représentent un pont substitué par un ou plusieurs atomes de fluor, qui avec deux atomes de carbone voisins du cycle benzénique de la formule (I) forme un cycle à cinq, six ou sept chaînons, qui est carbocyclique ou peut contenir des hétéroatomes tels que O, S ou N.

3. Utilisation selon la revendication 1, dans laquelle
A représente le groupe fluorométhyle, trifluorométhyle, 2-fluoroéthyle, 2,2,2-trifluoroéthyle, pentafluoroéthyle, 2,2,3,3-tétrafluoropropyle, perfluorobutyle, perfluoro-octyle ou les radicaux alcoxy correspondants, en particulier le groupe trifluorométhyle ou trifluorométhoxy et
B représente H,
ou A et B représentent ensemble -OCF₂O-, -OCF₂CF₂O-, -OCHFCHFO- ou -OCF₂CF₂CF₂O-.

4. Utilisation selon la revendication 1, **caractérisée en ce qu'**on utilise comme pigment de formule (I) au moins un pigment de formules (II) à (VIII),

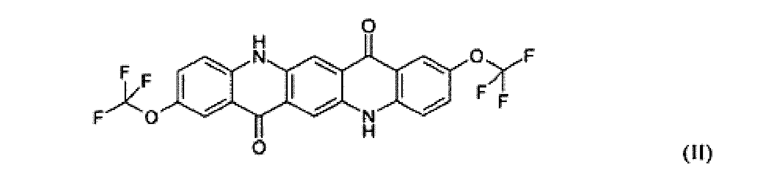

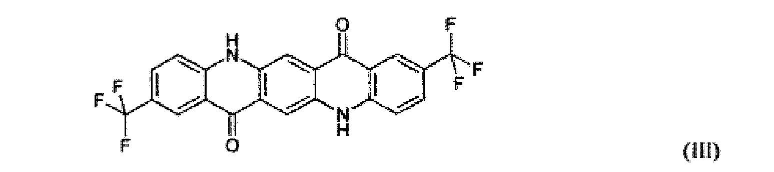

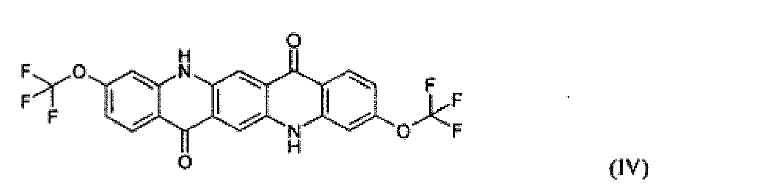

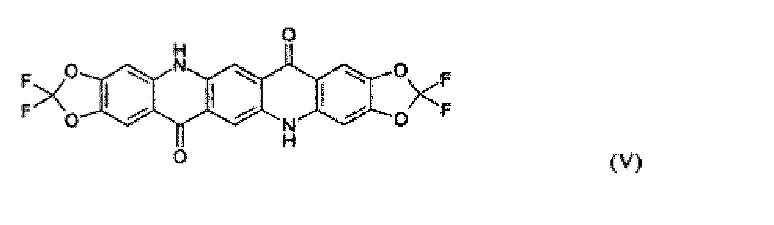

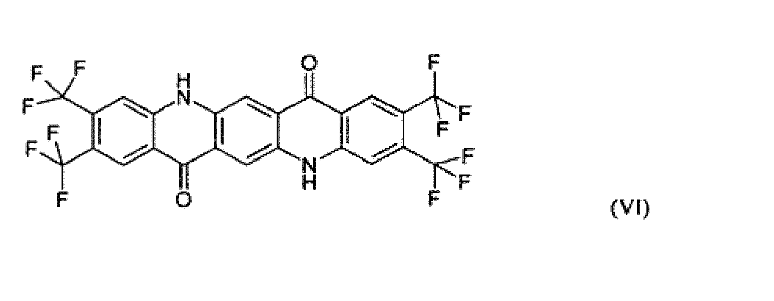

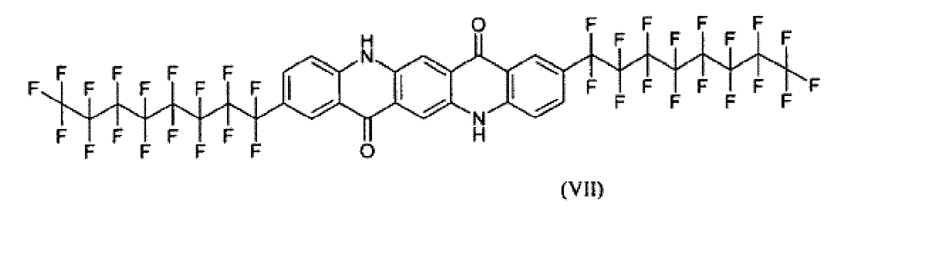

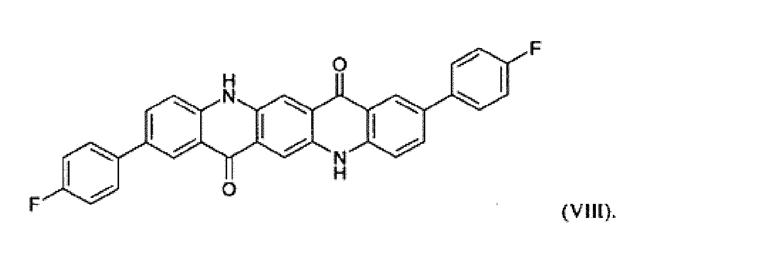

5. Utilisation selon au moins l'une quelconque des revendications 1 à 4, **caractérisée en ce qu'**on utilise le pigment de formule (I) en association avec d'autres pigments.

6. Utilisation selon la revendication 1, **caractérisée en ce que** le pigment de formule (I) présente une surface selon B.E.T de 40 - 200 m²/g.

7. Utilisation selon la revendication 1, **caractérisée en ce que** le pigment de formule (I) présente une facilité de dispersion de 10 à 500, mesurée selon DIN 53775, section 7, la température du calandrage à froid étant de 25 °C et la température du calandrage à chaud étant de 150 °C.

8. Photoresist, contenant au moins un monomère photodurcissable, au moins un photoamorceur et au moins un pigment de formule (I), tel que défini dans l'une quelconque des revendications 1 à 7.

9. Filtre coloré, contenant au moins un pigment tel que défini dans l'une quelconque des revendications 1 à 7.

10. Afficheur à cristaux liquides, contenant au moins un filtre coloré selon la revendication 9.

11. Composé de formule (II) 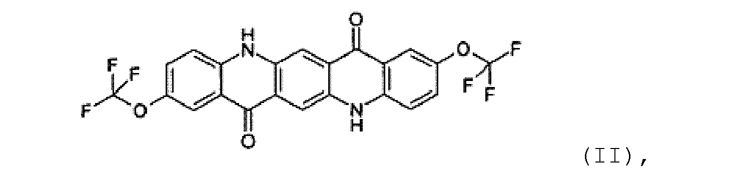 qui présente dans le diagramme de diffraction des rayons X (raie K_{α} du Cu) des lignes aux valeurs d suivantes :
d : 4,22 ; d : 3, 55 ; d : 3, 31 (forme B)

12. Composé de formule (IVa) 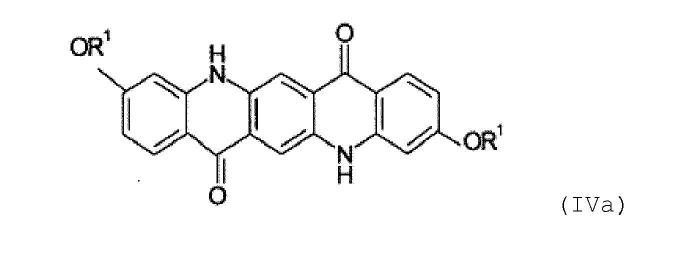 dans laquelle R¹ est un radical aliphatique fluoré, en particulier un radical en C₁-C₈ fluoré, de préférence le groupe fluorométhyle, trifluorométhyle, 2-fluoroéthyle, 2,2,2-trifluoroéthyle, pentafluoroéthyle, 2,2,3,3-tétrafluoropropyle, perfluorobutyle ou perfluoro-octyle, en particulier un composé de formule (IV)

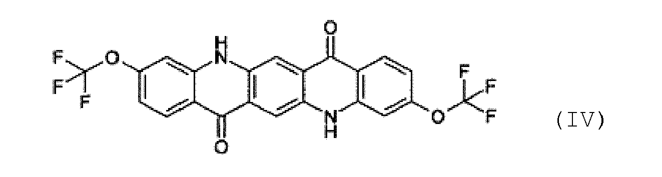

13. Composé de formule (Va) 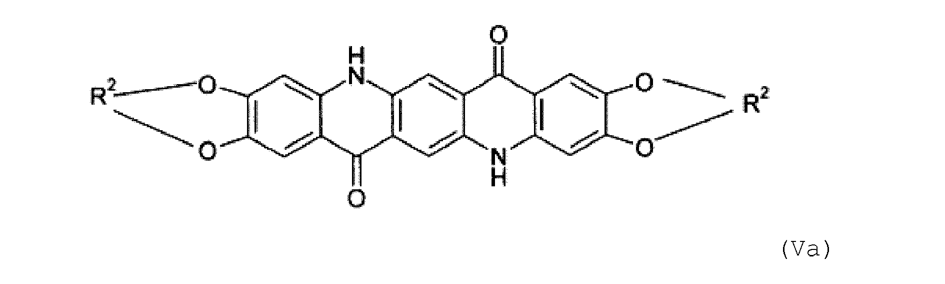 dans laquelle R² représente une unité alkylène en C₁-C₃ substituée par le fluor, en particulier -CF₂-, -CF₂CF₂-, -CHFCHF- ou -CF₂CF₂CF₂-, en particulier un composé de formule (V)

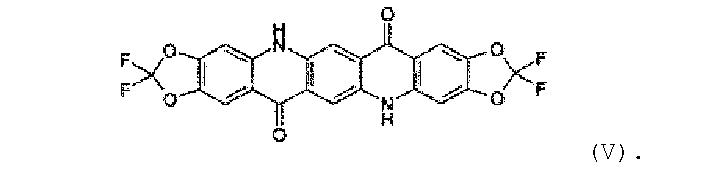

14. Mélanges, contenant
- au moins un pigment de formule (I),
- au moins un autre pigment rouge choisi dans le groupe constitué par C.I. Pigment Rouge 122, C.I. Pigment Rouge 149, C.I. Pigment Rouge 177, C.I. Pigment Rouge 179, C.I. Pigment Rouge 254 et C.I. Pigment Violet 19.

15. Mélanges, contenant
- au moins un pigment de formule (I),
- au moins un pigment jaune ayant une bande d'absorption dans la plage de 400 à 520 nm,
en particulier des pigments jaunes choisis dans le groupe constitué par C.I. Pigment Jaune 138, C.I. Pigment Jaune 139, C.I. Pigment Jaune 150, et un pigment complexe nickel-acide azobarbiturique 1:1 à mélamine intercalée.
